# EUROPEAN PATENT APPLICATION

(11) **EP 2 016 991 A1**
(43) Date of publication of application: **21.01.2009**
(21) Application number: 07112575.1
(22) Date of filing: 16.07.2007
(51) Int. Cl.: B01D 53/14, B01D 53/96, C07C 7/11, C07C 7/152, C07C 7/156, C07C 11/04

(54) **Processes for separation of gases using ionic liquids**

(71) Applicant: Nederlandse Organisatie voor Toegepast- Natuurwetenschappelijk Onderzoek TNO, 2628 VK Delft (NL)
(72) Inventor: van Erkel, Joost, 7314 CN, Apeldoorn (NL); Bressers, Petrus Marinus Martinus Cornelus, 8032 MZ, Zwolle (NL); Creusen, Raymond Johannes Maria, 3822 VE, Amersfoort (NL)
(74) Representative: van Loon, C.J.J.

(57) **Abstract**

The invention is directed to a process using a composition comprising at least one type of ionic liquid and at least one metal ion in separation processes, in particular separation processes wherein olefins and paraffins are separated.

## Description

The invention is in the field of separation processes, such as processes for concentrating gases from mixtures of gases.

Separation of individual gas components from gas mixtures comprising them is a very important process step in the chemical industry, in particular in petrochemical industry. A typical example is the separation of ethylene from a mixture comprising ethane and ethylene, which is difficult to perform as a result of the physical properties of both components, in particular boiling temperature, which are nearly identical. By result, separation of ethane/ethylene mixtures by the current state of the art (cryogenic distillation) requires a considerable amount of energy. Similar considerations may apply for the separation of other olefin/paraffin mixtures, or certain other gas mixtures. An overview of olefin/paraffin separation technologies is given by R.B. Eldridge (Ind. Eng. Chem. Res. 32 (1993) 2208-2212). In this work several types of separation processes are reviewed, including distillation, physical adsorption, physical absorption, chemical adsorption and chemical absorption.

Furthermore, D.J. Safarik and R.B. Eldridge (Ind. Eng. Chem. Res. 37 (1998) 2571-2581) discuss olefin/paraffin separations by chemical absorption (also called reactive absorption, facilitated transport or reversible chemical complexation). In contrast to distillation techniques, in which energy is used as a separating agent, in chemical absorption based methods, reversible chemical complexation is used to separate gas mixtures. Chemical absorption provides a selective and potentially low cost method for separation of gas mixtures.

In a process based on facilitated transport, the gas mixture is contacted with a liquid phase which carries a reagent that selectively and reversibly complexes the component of interest. After separation of the phases, the weak chemical interaction is reversed to regenerate the reactive reagent by displacement by another species, by temperature swings (higher temperature), and/or by pressure swings (lower pressure). It is desirable to use a complexing agent that forms a stable yet reversible complex. The interaction must however be weak enough to permit regeneration in an economically acceptable manner. The complexing agent must be inert towards undesired components and thermally stable to avoid irreversible loss. Finally, rates of complexation must be rapid to minimize contactor size.

Reversible chemical complexation is employed on an industrial scale for the purification of gas. Removal of CO₂ and H₂S from gas streams with amine solutions is a common industrial process.

In the art, separation of olefin/paraffin gas mixtures such as ethane/ethylene is for instance carried out using aqueous solutions of transition metal ions, such as copper (I) or silver (I) ions, which ions form electron donor/acceptor complexes with olefins.

Regeneration of the complexing agent is also possible using electrochemical cycling between the redox states of the complexing agents. By electrochemically changing the oxidation state from the desired state for complexation (for instance Cu(I)) to a non complexing oxidation state (for instance Cu(II)), the interaction with the desired gas can be modulated. This type of process is also referred to as electrochemically modulated complexation (EMC-process). EMC-processes are studied for several gas separations from gas mixtures.

The advantage of such a system is that the regeneration can take place without a pressure or temperature swing, which potentially saves costs. This way it is possible to extract the gas at low temperatures and if desired at a higher pressure, which saves compression costs.

Although with these known separation techniques gas mixtures, such as olefin/paraffin mixtures, can be separated successfully, the amount of solution that is required is considerable, in particular as a result of the low solubility of the metal salts, and the product obtained needs to be dried to remove water therefrom. As a result of these and other factors, these known processes are not employed in practice yet.

It is an object of the present invention to provide for another separation process, in particular a process that employs a different medium as the extractant. The process of the present invention aims at providing an improvement over the prior art techniques, in particular with respect to energy consumption.

It was found that this object can be met by using a complexing agent that is present in an ionic liquid. This mixture of complexing agent and ionic liquid is also referred to as the electrolyte. An ionic liquid is a liquid that comprises ions, without requiring the presence of a solvent, such as water..

An ionic liquid comprises a salt that is liquid under the process conditions, such as a melt of a salt. In general an ionic liquid used in a method of the invention, has a melting point below 200 °C, preferably of 100 °C or less, in particular of 50 °C or less. It is in particular preferred that the ionic liquid is liquid at or below 25 °C. Such liquid may be referred to as a room temperature liquid salt.

Surprisingly, the metal ions maintain their capacity to form complexes with the components to be extracted from the gas mixtures when present in ionic liquids.

It was found that the use of ionic liquids in combination with the metal ions provides for a solution that has a much higher concentration in metal ions than the concentration that would be possible using for instance water as the solvent.

The ionic liquid solutions of the present invention can be used to perform separation processes, which may be performed at a much faster operating speed than processes based on conventional (*viz*. aqueous) solutions, due to the higher concentrations of the metal ions.

Due to the very high electrochemical stability of ionic liquids (as compared to processes based on water and organic solvents) the process of the present invention is much more stable with less undesired side reactions, which conventionally may occur due to electrochemical decomposition of the electrolyte solution.

The processes of the present invention using the ionic liquid solutions of the present invention can be carried out in equipment that is considerably smaller than equipment that is employed using conventional solutions.

According to the present invention a product gas can be obtained that is essentially dry (*e*.*g*. having a water content as low as 1 wt.% or even less). Because of the very low vapour pressure of the ionic liquid (at room temperature in the order of magnitude of less than 10⁻⁵ kPa) the product gas can be obtained without impurities. This is highly desirable because an extra drying or purification step is no longer necessary.

The process of the present invention, using the ionic solutions of the present invention can be carried out in larger temperature window, depending on the type of ionic liquid used for the solutions of the present invention. For instance, ionic liquids are known that are liquid from temperatures as low as 0 °C or less, up to 300 °C, or even more.

Suitable salts that form an ionic liquid can for instance be chosen from the salts of the following cations, anions and from combinations thereof.

The cations may for instance be selected from cations with substituents having C₁-C₁₄ alkyl groups, in particular C₁-C₄ alkyl groups. Particularly suitable are cations selected from the group of monosubstitued imidazolium derivatives, disubstituted imidazolium derivatives, tri-substitued imidazolium derivatives, pyridinium derivatives, pyrrolidinium derivatives, phosphonium derivatives, ammonium derivatives, guanidinium derivatives and isouronium derivatives, including combinations thereof.

The anions may in particular be chosen from the group of chloride, bromide, iodide, nitrate, nitrite, fluoride, phosphate, imide, amide, borate, tosylate, tetrafluoroborate, hexafluoroborate, hexafluorophosphate, trifluoromethanesulfonate, methylsulfate, bis(pentafluoroethyl)phosphinate, thiocynate, octylsulfate, hexylsulfate, buthylsulfate, ethylsulfate, dicyanamide, hexafluoroantimonate, bis-(pentafluoroethyl)phospinate, bis-(trifluoromethyl)imide, trifluoroacetate, bis-trifluorsulfonimide, triflate and dicyanamide, including combinations thereof.

The metal ions used in the present invention are preferably transition metal ions, more preferably Cu⁺, Ag⁺, Ni⁺, Pt⁺ or combinations thereof. The metal ions can be added to the ionic liquid in the form of a salt. The anions of the metal salt can be chosen from the group of chloride, bromide, iodide, nitrate, nitrite, fluoride, phosphate, imide, amide, borate, tosylate, tetrafluoroborate, hexafluoroborate, hexafluorophosphate, trifluoromethanesulfonate, methylsulfate, bis(pentafluoroethyl)phosphinate, thiocynate, octylsulfate, hexylsulfate, buthylsulfate, ethylsulfate, dicyanamide, hexafluoroantimonate, bis-(pentafluoroethyl)phospinate, bis-(trifluoromethyl)imide, trifluoroacetate, bis-trifluorsulfonimide, triflate and dicyanamide, including combinations thereof.

In particular the following redox couples can be used:
For Cu and Ni two redox couples can be used:

Cu⁺⁺ + e- ←→ Cu⁺ or Cu⁺ + e- ←→ Cu(s)

Ni⁺⁺ + e- ←→ Ni⁺ or Ni⁺ + e- ←→ Ni(s)

For Ag and Pt only one redox couple is applicable:

Ag⁺ + e- ←→ Ag(s)

Pt⁺ + e- ←→ Pt(s)

For example, ethylene can form a complex with the monovalent entities.

The process of the present invention typically comprises the following steps.

In a first step the gas mixture (*e*.*g*. ethylene/ethane) is contacted with the ionic liquid by which the bonding compound (*e*.*g*. ethylene) is (preferentially) bonded to the metal ions. This contacting can be carried out in various ways, *e*.*g*. using conventional absorbers, gas columns, gas scrubbers and/or membrane gas absorbers. After a sufficient amount of compound is bonded to the metal ion, the solution is separated from the gas phase, wherein the liquid phase is enriched in compound bonded to the metal ions and the gas phase is enriched in the non-bonding compound (*e*.*g*. ethane).

In a second step the metal ions are converted electrochemically to an oxidation state which causes the bonding between the bonding compound (*e*.*g*. ethylene) and the metal ion to be broken.

For instance, copper can be oxidized on the anode: Cu⁺ → Cu⁺⁺ + e⁻. To prevent the anode from dissolving, it has to be sufficiently inert. Suitable materials are Pt, Ag and Au, which ensures that only Cu oxidizes. Similarly, nickel can be oxidized on an anode.

Silver yields reduction on the cathode: Ag⁺ +e- → Ag. Thus deposition of silver is obtained. The same applies to platinum.

In a third step, the bonding compound (*e*.*g*. ethylene) is separated from the ionic liquid by which the compound can be recovered as a purified product stream. According to the invention this step can be carried out at a relatively low temperature and/or higher pressure. This provides possible cost reduction on the costs of compression. This step can be carried out using conventional techniques, *e*.*g*. using desorbers, gas colums, gas scrubbers and/or membrane gas desorbers.

In a fourth step of the process of the present invention the metal ions are electrochemically converted back into *e*.*g*. into monovalent oxidation state, which is the oxidation state that has the highest affinity for complex forming with the compound to be absorbed (*e*.*g*. an olefin, such as ethylene). Thus copper reduces on the cathode: Cu⁺⁺ + e- → Cu⁺. Suitable cathodes are based on Pt, Ag or Au.

Silver oxidation occurs on a silver anode. Thus preferably a silver electrode or an electrode comprising a silver layer is used:Ag → Ag⁺ +e⁻. As a result silver is dissolved. Again nickel is comparable to copper: Ni⁺⁺ + e- →Ni⁺; and platinum is comparable to silver.

Silver and platinum yield deposition in Step 2 and dissolution in Step 4. In Step 2 the electrode gains mass, which it loses in Step 4. For a continuous process in accordance with the present invention, the polarity can be changed at certain intervals. Pt is generally not a suitable choice in aqueous and organic solvents, because Pt cannot be oxidized. However, in ionic liquids it is possible due to the higher electrochemical stability, since ionic liquids oxidize only at very high potential differences.

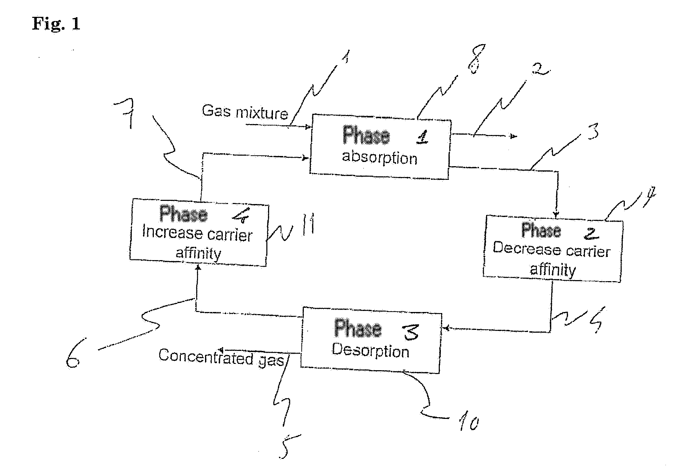

The process is preferably carried out in a continuous fashion, *e*.*g*. as schematically illustrated in Figure 1. According to the process of Figure 1, gas mixture (1) is contacted in absorber (8) with the solution of the invention, comprising the ionic liquid and the metal ions. Stream (2) is a stream that is depleted in the non-absorbing component (*e*.*g*. paraffin, such as ethane). The solution is charged by the absorbing compound (*e*.*g*. olefin, such as ethylene) is obtained as stream (3), which is fed to an electrochemical reactor (9), where the affinity between metal ion and absorbed compound is decreased. Subsequently, liquid product stream (4) is fed to the desorber (10), where the gas phase is separated from the liquid phase. The desorbed gas (*e*.*g*. olefin, such as ethylene) is obtained as a separate concentrated gas stream (5). The liquid stream (6) is fed to an electrochemical reactor (11) where it regains its affinity. Subsequently, liquid stream (7) having the proper affinity again is fed again to the absorber (2). Optionally a vent (not shown) may be applied in the liquid circuit to avoid build-up of contamination in the liquids stream. It is also possible to construct the two electrochemical units in one single electrochemical cell, by separating the half-cells from each other by means of a membrane.

Although the invention has been elucidated hereinabove by referring to separation of mixtures of olefins and paraffins, in particular ethylene and ethane, it is not limited to separating mixtures of these compounds. Other gas mixtures that can be separated in accordance with the present invention are for instance CO₂ comprising mixtures, such as CO₂/CH₄ or CO₂/N₂; CO₂/flue gas; NO comprising mixtures such as flue gases; CO comprising mixtures, such as flue gases; H₂S comprising mixtures, such as off-gases from fermentation processes. CO₂ and H₂S comprising mixtures may suitably be separated using compounds having amine groups. These compounds can be added in accordance with the present invention in the form of salts and/or solvents, which are dissolved in the ionic liquid. It is also possible to use quinines for this purpose.

CO is suitably complexed by Cu(I), see *e*.*g*. Terry et al. (AIChE Journal; 41(1995)2556-2564.

NO can suitably be complexed by Fe(II).

The present invention will now be illustrated by the following examples.

### Example 1

Electrochemical desorption of an ethane loaded ionic liquid containing Ag⁺ ions.

The electrolyte used comprised 1-ethyl-3-methyl-imidazolium bis[(trifluoroomethyl)sulfonyl]amide with 20 mol% silver bis[(trifluoroomethyl)sulfonyl]amide. The electrolyte was vacuum dried at 80 °C for 16 hours prior to use.

Then the electrolyte solution (20 ml) was loaded with ethane at 20 °C for 16 hours to reach saturation with ethane by allowing the ethane gas to bubble through the solution at an overpressure. This was carried out in a vessel. After reaching equilibrium, the vessel was closed and the pressure above the electrolyte solution was measured (free volume inside the vessel approximately 180 ml). The silver ions in the stirred electrolyte solution were potentiostatically reduced on a silver working electrode at -1.0 V with respect to an Ag/Ag⁺ reference electrode for approximately 3000 seconds. The total reduction charge used for the silver reduction was approximately 15 Coulombs. During the electrochemical reduction process the pressure inside the vessel remained stable showing no pressure increase.

### Example 2

Electrochemical desorption of an ethylene loaded ionic liquid containing Ag⁺ ions.

The same electrolyte as in Example 1 was vacuum dried as described in Example 1. Again 20 ml of the electrolyte transferred to a vessel and this time it was loaded with ethylene at 20 °C for 16 hours to reach saturation with ethylene in a similar fashion as in Example 1. After reaching the equilibrium, the vessel was closed and the pressure above the electrolyte solution was measured (free volume inside the vessel approximately 180 ml). The silver ions in the stirred electrolyte solution were potentiostatically reduced on a silver working electrode at -1.0 V with respect to an Ag/Ag⁺ reference electrode for approximately 1650 seconds. The total reduction charge used for the silver reduction was approximately 12 Coulombs. During the electrochemical reduction process the pressure inside the vessel remained steadily increased. The total pressure increase at the end of the reduction process was 50 mbar. Upon ending the reduction process the pressure increase stopped.

## Claims

1. Process for separating a gas mixture of two or more components comprising the steps of:
*i*. contacting said gas mixture with a mixture comprising an ionic liquid and at least one metal ion, whereby at least one bonding component bonds with said metal ion;
*ii*. converting electrochemically the oxidation state of said metal ion;
*iii*. separating the at least one bonding component from the ionic liquid; and
iv. converting back said metal ions into the state they had in step *i*.

2. Process according to claim 1, wherein said mixture comprising an ionic liquid comprises an ionic liquid selected from imidazolium derivatives, pyridinium derivatives, and combinations thereof.

3. Process according to any of the previous claims, wherein said metal ion is a transition metal ion.

4. Process according to claim 3, wherein the metal ion is selected from Cu, Ag, Ni, Pt and combinations thereof.

5. Process according to any of the previous claims, wherein said mixture comprising an ionic liquid comprises cations that are selected from cations with substituents having C₁-C₁₄ alkyl groups, preferably C₁-C₄ alkyl groups.

6. Process according to claim 5, wherein the cations are selected from the group of monosubstitued imidazolium derivatives, disubstituted imidazolium derivatives, tri-substitued imidazolium derivatives, pyridinium derivatives, pyrrolidinium derivatives, phosphonium derivatives, ammonium derivatives, guanidinium derivatives, isouronium derivatives, and combinations thereof.

7. Process according to any of the previous claims, wherein said mixture comprising an ionic liquid comprises anions that are selected from the group of chloride, bromide, iodide, nitrate, nitrite, fluoride, phosphate, imide, amide, borate, tosylate, tetrafluoroborate, hexafluoroborate, hexafluorophosphate, trifluoromethanesulfonate, methylsulfate, bis(pentafluoroethyl)phosphinate, thiocynate, octylsulfate, hexylsulfate, buthylsulfate, ethylsulfate, dicyanamide, hexafluoroantimonate, bis-(pentafluoroethyl)phospinate, bis-(trifluoromethyl)imide, trifluoroacetate, bis-trifluorsulfonimide, triflate, dicyanamide, and combinations thereof.

8. Use of a mixture comprising an ionic liquid as defined in any of the previous claims in a separation process.

9. Use according to claim 8, wherein said separation process comprises the separation of an olefin and a paraffin.
